# EUROPÄISCHE PATENTANMELDUNG

(11) **EP 2 420 328 A1**
(43) Veröffentlichungstag der Anmeldung: **22.02.2012**
(21) Anmeldenummer: 11006344.3
(22) Anmeldetag: 02.08.2011
(51) Int. Cl.: B09B 3/00, B03B 9/06

(54) **Verfahren und Vorrichtung zur Behandlung eines organik- und wasserhaltigen Stoffgemisches**

(30) Priorität: 16.08.2010 DE 102010034528
(71) Anmelder: Schalk, Peter, Dr., 79331 Teningen (DE)
(72) Erfinder: Schalk, Peter, Dr., 79331 Teningen (DE)
(74) Vertreter: Otten, Roth, Dobler & Partner

(57) **Zusammenfassung**

Die Erfindung betrifft ein Verfahren und Vorrichtung zur Behandlung eines organik- und wasserhaltigen Stoffgemisches.

## Beschreibung

"Verfahren und Vorrichtung zur Behandlung eines organik- und wasserhaltigen Stoffgemisches"

Die Erfindung betrifft ein Verfahren und eine Anlage zur Behandlung eines organik- und wasserhaltigen Stoffgemisches nach dem Oberbegriff der Ansprüche 1 bis 15.

### BESCHREIBUNG

Die mechanische und biologische Behandlung und Verwertung von organikhaltigen Stoffgemischen unterschiedlicher Herkunft, Biomassen und Abfällen gewinnt weltweit an Bedeutung. Dabei stehen die stoffliche Verwertung wie auch die Energieerzeugung aus den erneuerbaren Energiequellen (z. B. Biomassen) sowie die Verhinderung von Umweltbelastungen aus Abfällen im Vordergrund.

Dem biogenen Anteil in den Stoffgemischen wird vielfach eine besondere Aufmerksamkeit gewidmet. Die biogenen Abfälle haben einen hohen Wassergehalt, was sowohl die biologische Behandlung (Kompostierung, Vergärung) als auch eine thermische Behandlung (Verbrennung) nachteilig beeinträchtigt. Bei der Deponierung sind die biogenen Abfälle die wesentliche Schadstoffquelle für Emissionen (Deponiegase, Deponiesickerwasser). Der biologische Abbau und insbesondere die Entwässerung reduzieren die organischen (z. B. Kohlenstoffverbindungen) und anorganischen (z. B. Stickstoff) Schadstoffe. Insbesondere die Entwässerung verringert in erheblichem Umfang die Reststoffmenge, die weiterzubehandeln ist.

### STAND DER TECHNIK

Hierzu wird beispielsweise auf Verfahren der sogenannten Trockenfermentation unter Berieselung der Abfalls mit Kreislaufwasser (Perkolation) verweisen. Dabei nimmt der Abfall erhebliche Wassermengen auf, die in der anschließenden Nachrotte vielfach zu Schwierigkeiten führen. Darüber hinaus erfolgt in der Trockenfermentation weder eine Durchmischung oder Umwälzung des Abfalls im Reaktor noch eine aktive Entwässerung des Gärrestes. Die Biogasausbeuten dieser Verfahren liegen deshalb deutlich unterhalb von Nassfermentationssystemen. Im Ergebnis verlassen mit dem Gärrest erhebliche Mengen nicht abgebauter organischer Substanzen die Vergärung. Desweiteren verlangt die aerobe Nachbehandlung (Kompostierung) eine Wassergehalt von max. 60 Gewichtsprozent sowie ein ausreichend be- und entlüftetes Haufwerk. Die hohen Wassergehalte von bis zu 80 Gewichtsprozent sowie die Struk=urschwäche des Gärrestes aus der Trockenfermentation bereiten vielfach Schwierigkeiten.

Viele Verfahren der Abfallbehandlung haben keine oder eine unzureichende Entwässerung nach der Vergärung sowie Behandlung von Prozesswasser bzw. Abwasser. Da aber das Wasser und seine Inhaltsstoffe bei der Abfallbehandlung eine entscheidende Rolle in den Prozessschritten Abfall-Wasser-Biogas sowie in der Mengenbilanz und Schadstoffbelastung einnehmen, ist die Prozess- und Abwasserreinigung in das Abfallbehandlungskonzept zu integrieren. Die Entwässerung von mechanisch vorbehandelten Abfällen bzw. Stoffgemischen oder Gärresten aus der Vergärung liefert einerseits zusätzliches Biogas und andererseits ein entwässertes Reststoffgemisch, das einfacher biologisch oder thermisch nachbehandelt werden kann.

### AUFGABEN UND VORTEILE DER ERFINDUNG

Der Erfindung liegt die Aufgabe zugrunde, ein verbessertes Verfahren und eine verbesserte Anlage zur mechanischen und biologischen Behandlung eines organik- und wasserhaltigen Stoffgemisches, das biologisch umsetzbare Stoffe sowie stofflich und/oder energetisch verwertbare Fraktionen enthält. Hieraus ergibt sich eine äußerst effektive und kostengünstige Behandlungsmethode zur Erhöhung der Biogasausbeute in der Vergärung, zur Massenreduzierung der Reststoffe und Vorbehandlung für eine aerobe Stabilisierung (z.B. Trocknung, Nachkompostierung) oder Verbrennung des Reststoffgemisches. Diese Aufgabe wird durch die Merkmale der Verfahrens und Anlagenansprüche 1 bis 15 gelöst.

Die Erfindung bedient sich dabei einzelner, z. T. an sich bekannter Verfahrensschritte und Anlagen, die jedoch für die mechanische und biologische Behandlung des organik- und wasserhaltigen Stoffgemisches modifiziert werden und in ihrer Kombination zu einem effektiveren und optimierten Verfahren führen. Dabei werden die aeroben und anaeroben biologischen Vorgänge im Verfahren gefördert und beschleunigt. Die Behandlung des Stoffgemisches ist hierbei schwerpunktmäßig auf die Auswaschung und mechanische Entwässerung organikhaltiger Stoffgemische gerichtet. Die Auswaschung organischer und anorganischer Substanzen in Verbindung mit der mechanischen Entwässerung erhöht die Biogasausbeute um bis zu 30 %. Darüber hinaus lässt sich das Reststoffgemisch einer einfacheren Nachbehandlung (z. B. Kompostierung, Trocknung, Verbrennung) unterziehen. Beispielsweise wird der mikrobielle Aufschluss (Hydrolyse) im Gegensatz zu anderen Vergärungsverfahren nicht im Abfallstoffgemisch vorgenommen, sondern im erzeugten Prozessproduktwasser aus der Auswaschung und Entwässerung. Hieraus ergeben sich wesentliche Vorteile in der Begünstigung der mikrobiellen Prozesse bzgl. einer höheren Intensität und einer rascheren Umsetzung. Das organik- und wasserhaltige Stoffgemisch hat hierbei vergleichsweise eine äußerst kurze Kontaktzeit, so dass auch verhältnismäßig hohe Anlagendurchsätze erzielt werden können.

Das Prozessproduktwasser wird für die einzelne Behandlungsstufe mechanisch und/oder biologisch und/oder chemisch-physikalisch aufbereitet, um für die jeweilige Prozessstufe vorteilhafte Eigenschaften zu erlangen. In besonderer Weise können auch Abfallstoffgemische behandelt werden, die eine hohe organische und/oder nasse Stofffraktion aufweisen und deshalb mit Bezug auf den für die aerobe Stabilisierung erforderlichen Strukturerhalt im Stoffgemisch einer besonders schonenden mechanischen Behandlung bedürfen. Mit der Auswaschung und Entwässerung wird eine effiziente Trennung des Stoffgemisches in Wasserpfad (Aufbereitung des Prozessproduktwassers) und Feststoffpfad (Reststoffgemisch zur Weiterbehandlung) vorgenommen.

Des weiteren hat die vorliegende Erfindung den Vorteil, dass aus einem höchst unterschiedlichen Eingangsprodukt ein Endprodukt geschaffen wird, welches ordnungsgemäß weiterbehandelt und verwertet werden kann. Dies gilt für unbehandelte, mechanisch vorbehandelte aber auch biologisch vorbehandelte Stoffgemische.

Unter dem Begriff "Stoffgemisch" sind im Sinne der vorliegenden Erfindung organik- und wasserhaltige Stoffgemische aus Haushalts- und Siedlungsabfällen, Grünschnitt, Bioabfälle (z.B. Küchenabfälle, Lebensmittelproduktionsabfälle), Gewerbeabfälle, land- und forstwirtschaftliche Biomassen, Altlasten aus Abfalldeponien oder dergleichen zu verstehen. Bei den Stoffgemischen kann es sich um unbehandelte (ohne mechanische Vorbehandlung), mechanisch vorbehandelte aber auch biologisch vorbehandelte (aus z. B. Kompostierung, Vergärung) Stoffgemische handeln.

Unter dem Begriff "Prozesswasser" ist im Sinne der vorliegenden Erfindung Wasser oder Flüssigkeit einschließlich gelöster und/oder ungelöster Stoffe zu verstehen, das/die in den Prozessstufen verwendet wird.

Unter dem Begriff "Prozessproduktwasser" ist im Sinne der vorliegenden Erfindung Wasser oder Flüssigkeit einschließlich gelöster und/oder ungelöster Stoffe zu verstehen, das/die aus den Prozessstufen entsteht.

Die Erfindung wird anhand von Ausführungsbeispielen näher erläutert, die in den Zeichnungen dargestellt und in der nachfolgenden Beschreibung näher erläutert sind. Dabei zeigen
- Fig. 1:: eine schematische Darstellung des erfindungsgemäßen Verfahrens bzw. der erfindungsgemäßen Anlage,

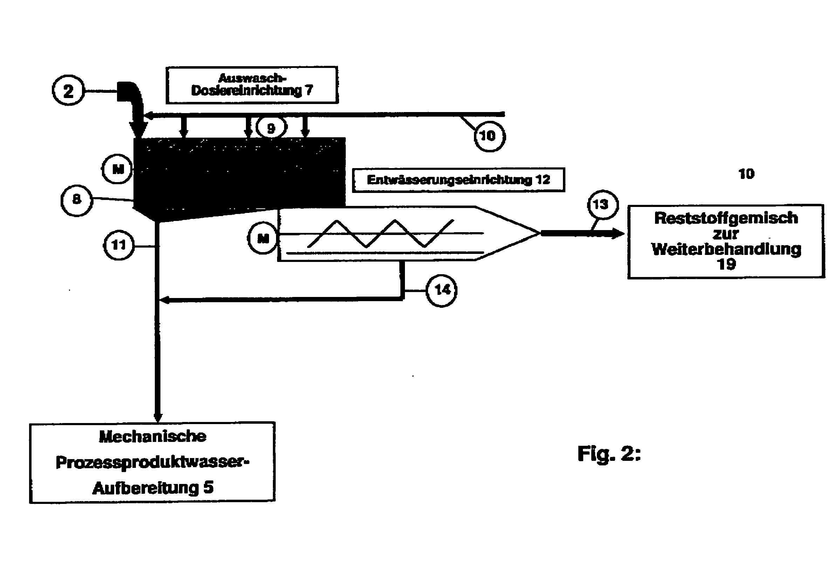

- Fig. 2:: eine detaillierte Darstellung des erfindungsgemäßen Verfahrens bzw. der erfindungsgemäßen Anlage

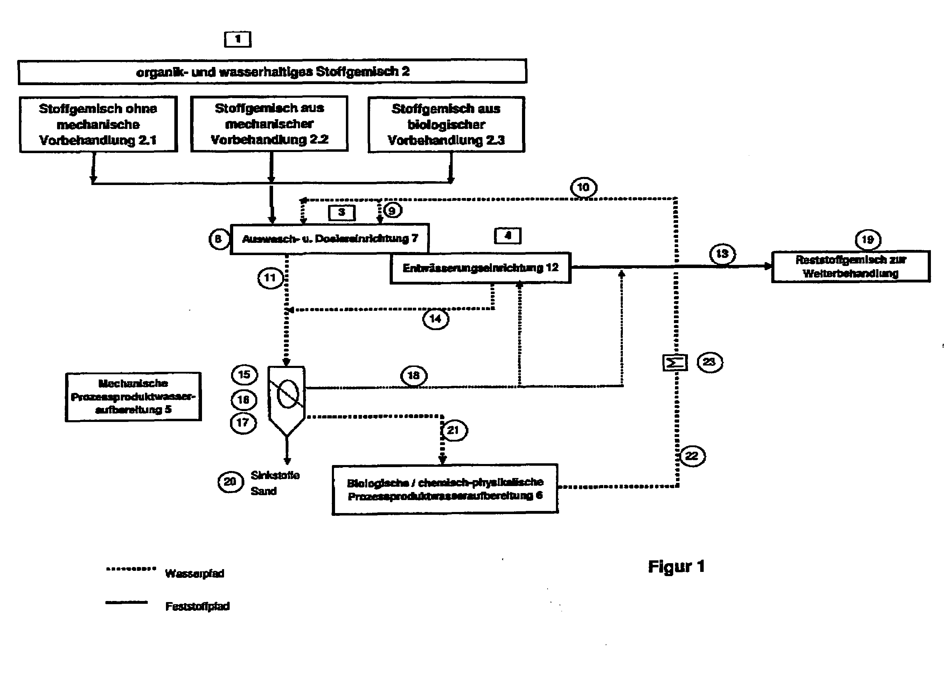

Eine in der Fig. 1 schematisch dargestellte Anlage 1 dient zur Behandlung mittels Auswaschung und mechanischen Entwässerung eines Stoffgemisches 2 (2.1; 2.2; 2.3) unterschiedlicher Herkunft bzw. Vorbehandlung aus Siedlungs- und/oder Gewerbeabfällen und/oder Biomassen, die u.a. organische biologisch umsetzbare Stoffe sowie stofflich und/oder energetisch verwertbare Fraktionen enthalten. Eine mechanische Vorbehandlung des Stoffgemisches 2.2 vor dem erfindungsgemäßen Verfahren richtet sich nach der Zusammensetzung des zu behandelnden Stoffgemisches sowie den Zielsetzung der Behandlung. Üblicherweise erfolgt die mechanische Vorbehandlung über Dekompaktierung, Zerkleinerung, Siebung, Sichtung und Entnahme der Wert- und Störstoffe. Es kann aber auch auf die mechanische Vorbehandlung des Stoffgemisches 2.1 in bestimmten Fällen verzichtet werden. Die Anlage 1 kann aber auch ein organik- und wasserhaltiges Stoffgemisch 2.3 aus einer biologischen Vorbehandlung verarbeiten. Es handelt sich hierbei um den Produktfeststoff aus einer Kompostierung oder Vergärung (Gärrest).

Dabei umfasst die Anlage 1 zur Auswaschung und mechanischen Entwässerung eines Stoffgemisches 2 (im Folgenden identisch mit 2.1; 2.2; 2.3) im wesentlichen zum Beispiel eine erste Prozessstufe 3 und eine zweite Prozessstufe 4 für die Behandlung des organik- und wasserhaltigen Stoffgemisches 2. Die mechanische Aufbereitung 5 und biologische bzw. chemisch-physikalische Aufbereitung 6 des Prozessproduktwassers zu einem wiederverwertbaren Prozesswassers 10 kann in weiteren Behandlungsstufen 5 und 6 erfolgen.

In der ersten Prozessstufe 3 wird das zugeführte Stoffgemisch 2 einer intensiven Auswaschung mit teilweise Einstauung des Stoffgemisches (Perkolation) unterzogen. Die erste Prozessstufe 3 ist als Auswasch- und Dosiereinrichtung 7 ausgebildet und besteht aus einer dynamischen (rotierend) Auswascheinrichtung 7 und beispielsweise aus einer und/oder mehreren neben und/oder hintereinander angeordneten Schnecken oder Spiralen mit Siebeinrichtung 8, wobei das zu behandelnde Stoffgemisch 2 kontinuierlich oder diskontinuierlich der zweiten Prozessstufe 4 zugeführt wird. Bei der Siebeinrichtung 8 handelt es sich beispielsweise um eine Spalt-, Loch- oder/und Ringsieb mit einem Siebschnitt von vorzugsweise 5 - 12 mm. Die Auswasch- und Dosiereinrichtung 7 nimmt auch eine Entzerrung (Dekompaktierung) und mechanischen Aufschluss des Stoffgemisches 2 vor. Das sich in der Auswasch- und Dosiereinrichtung 7 befindende Stoffgemisch 2 wird über eine, sich über die gesamte Länge der Auswasch- und Dosiereinrichtung 7 erstreckende Berieselungseinrichtung 9 mit Prozesswasser als Auswaschflüssigkeit 10 behandelt. Dabei erfolgt die Behandlung derart, dass einerseits leicht lösliche organische und anorganische Stoffkomponenten und andererseits inerte abrasive Substanzen mit dem Prozesswasser 10 als Prozessproduktwasser 11 ausgetragen und der mechanischen Prozessproduktwasseraufbreitung 5 zugeführt werden. Unter "inerte Substanzen" bzw. "Inertstoffe" werden dabei biologisch nicht abbaubare Stoffe verstanden, das heißt z.B. mineralische Stoffe wie Sand.

Die Verweildauer des Stoffgemisches 2 in der Auswasch- und Dosiereinrichtung 7 beträgt ca. 0,1 bis 24 Stunden und vorzugsweise 2 bis 6 Stunden.

In der ersten 3 und zweiten 4 Prozessstufe erfolgt die Trennung des Stoffgemisches 2 in einen Wasserpfad und einen Feststoffpfad. Den Prozessstufen 3 und 4 folgt ein Wasserpfad, in dem weitere Prozessstufen 5 und 6 folgen können.

Das in der ersten Prozessstufe 3 behandelte Stoffgemisch 2 gelangt durch die Auswasch- und Dosiereinrichtung 7 zur zweiten Prozessstufe 4. Die zweite Prozessstufe 4 besteht aus einer mechanischen Entwässerungseinrichtung 12, die beispielsweise als Schneckenpresse mit Siebeinrichtung ausgeführt sein kann. Ein ausgewaschenes und entwässertes Reststoffgemisch 13 aus der Entwässerungseinrichtung 12 bietet als schadstoffentfrachteter und homogenisierter Feststoff die Voraussetzung für eine Weiterbehandlung 19 z.B. Kompostierung, Vergärung, Trocknung oder Verbrennung.

Das aus der Entwässerungseinrichtung 12 anfallende Prozessproduktwasser 14 ist angereichert mit gelösten und festen organischen Stoffe sowie Inertstoffen und wird der mechanischen Prozesswasserproduktaufbereitung 5 zugeführt. Die mechanische Prozesswasserproduktaufbereitung 5 behandelt auch das Prozessproduktwasser 11 aus der ersten Prozessstufe 3. In dieser Stufe 5 erfolgt eine Abtrennung von Feststoffen wie Inertstoffe, Grob- und Faserstoffe sowie Störstoffe, welche die nachfolgende Prozessproduktwasserbehandlung 6 beeinträchtigen können. Die mechanische Prozessproduktwasseraufbereitung 5 enthält beispielsweise Einrichtungen zur Schwimmdeckenräumung 15, einen Sandfang 16 für die Inertstoffabscheidung und Sieb- bzw. Trenneinrichtungen 17 für Grob- und Faserstoffe. Die abgeschiedenen Feststoffe 18 (z.B. Schwimm-, Faser-, Grobstoffe) aus der Schwimmdeckenräumung 15 und der Sieb- bzw. Trenneinrichtung 17 werden der zweiten Prozessstufe 4 zugeführt. Es besteht aber auch die Möglichkeit, die abgeschiedenen Feststoffe 18 dem Reststoffgemisch 19 zur Weiterbehandlung zuzuführen.

Die aus dem Sandfang 16 abgeschiedenen Inertstoffe wie Sand, Sinkstoffe 20 werden deponiert oder über eine weitergehende Aufbereitung verwertet.

Ein von Feststoffen 18 (z.B. Schwimm-, Faser-, Grobstoffe) sowie Inertstoffen 20 entfrachtetes Prozessproduktwasser 21 (aus 14 und 11) wird der biologischen und/oder chemisch-physikalischen Prozessproduktwasseraufbereitung 6 zugeführt. Die biologische Prozessproduktwasseraufbereitung 6 bedient sich herkömmlicher Aufbereitungsschritte und kann beispielsweise eine einstufige oder zweistufige Vergärung zur Erzeugung von Biogas sein. In der biologischen Prozessproduktwasseraufbereitung 6 kann aber auch eine aerobe Behandlung des Prozessproduktwassers 21 erfolgen. Hierbei werden Inhaltsstoffe des Prozessproduktwassers 21 oxidiert und Schadstoffe abgebaut. Eine weitere Möglichkeit ist die chemisch-physikalische Prozessproduktwasseraufbereitung 6 des Prozessproduktwassers 21. Hierbei werden aus dem Prozessproduktwasser 21 Inhaltsstoffe und ggf. Schadstoffe durch eine herkömmliche chemisch-physikalische Wasseraufbreitungstechnologie beispielsweise Flockung/Fällung oder Membranfiltration entnommen.

Die biologische / chemisch-physikalische Prozessproduktwasseraufbereitung 6 erzeugt ein Prozesswasser 22, das der Erwärmungsstufe 23 zugeführt. Diese Einrichtung dient der Erwärmung des zu verwendenden Prozesswasser 10 für die Auswasch- und Dosiereinrichtung 7 mindestens 35 °C, vorzugsweise 40-70°C. Mit der Erwärmung des Prozesswassers 10 wird in der ersten Prozessstufe 3 das Auswaschen und Herauslösen organischer und anorganischer Stoffkomponenten begünstigt. Des weiteren kann durch die Erwärmung eine Hygienisierung des zugeführten Stoffgemisches 2 vorgenommen werden. Die Wärmebereizstellung für die Erwärmungsstufe 23 kann aus der Verwertung des Biogases der biologischen Prozessproduktwasseraufbereitung 6 erfolgen.

Die Erfindung ist nichz auf die dargestellten und beschriebenen Ausführungsbeispiele beschränkt. Sie umfasst auch vielmehr alle Abwandlungen im Rahmen der Schutzrechtsansprüche.

### Bezugszeichenliste:

- 1: Anlage
- 2: Stoffgemisch einschließlich 2.1, 2.2 und 2.3
- 3: erste Prozessstufe
- 4: zweite Prozessstufe
- 5: Behandlungsstufe
- 6: Behandlungsstufe
- 7: Auswasch- und Dosiereinrichtung
- 8: Siebeinrichtung für 7
- 9: Berieselungseinrichtung für 7
- 10: Prozesswasser für 7
- 11: Prozessproduktwasser aus 7
- 12: Entwässerungseinrichtung
- 13: ausgewaschenes und entwässertes Reststoffgemisch
- 14: Prozessproduktwasser aus 12
- 15: Einrichtung zur Schwimmdeckenräumung
- 16: Sandfang
- 17: Sieb- und Trenneinrichtung für Grob- und Faserstoffe
- 18: abgeschiedene Feststoffe aus 5
- 19: Reststoffgemisch zur Weiterbehandlung
- 20: Sand, Sinkstoffe aus 5
- 21: Prozessproduktwasser aus 5
- 22: Prozesswasser aus 6
- 23: Erwärmungseinrichtung

## Patentansprüche

1. Verfahren (1) zur Behandlung eines organik- und/oder wasserhaltigen Stoffgemisches (2) insbesondere aus Abfällen und/oder insbesondere aus Biomassen, welches unbehandelt (2.1) und /oder mechanisch vorbehandelt (2.2) und/oder biologisch vorbehandelt (2.3) ist,
- wobei das Stoffgemisch (2) einer ersten Prozessstufe (3) zugeführt wird, in welcher mittels eines wenigstens teilweise wiederverwendeten Prozesswassers (10, 11, 14, 21, 22) in einer Auswasch- und Dosiereinrichtung (7) insbesondere eine intensive Auswaschung mit insbesondere teilweiser Einstauung des Stoffgemisches (2) sowie insbesondere eine Durchmischung, insbesondere eine Entzerrung und insbesondere eine kontinuierliche und/oder insbesondere eine diskontinuierliche Dosierung in eine zweite Prozessstufe (4) erfolgen;
- wobei das in der ersten Prozessstufe (3) behandelte Stoffgemisch (2) in der erwähnten zweiten Prozessstufe (4) insbesondere mittels einer mechanisch wirkenden Entwässerungseinrichtung (12) entwässert wird;
- wobei das Stoffgemisch (2) durch die erste Prozessstufe (3) und die zweite Prozessstufe (4) in einen Wasserpfad mit weiteren Behandlungsstufen (5, 6) für das Prozessproduktwasser (11, 14, 21) und einen Feststoffpfad zur Weiterbehandlung (19) des insbesondere ausgewaschenen und insbesondere entwässerten Reststoffgemisches (13) getrennt wird.

2. Verfahren nach Anspruch 1, **dadurch gekennzeichnet, dass** der Wasserpfad sowohl aus der ersten Prozessstufe (3) als auch aus der zweiten Prozessstufe (4) direkt mit Prozessproduktwasser (11, 14, 21) gespeist wird.

3. Verfahren nach einem der vorhergehenden Ansprüche, **dadurch gekennzeichnet, dass** das organik- und/oder wasserhaltige Stoffgemische (2) insbesondere aus Abfällen zusammengesetzt ist, welche
- insbesondere Haushalts- und Siedlungsabfällen insbesondere mit Grünschnitt und/oder insbesondere mit Bioabfällen wie insbesondere Küchenabfälle und Lebensmittelproduktionsabfällen und/oder
- insbesondere Gewerbeabfälle und/oder
und/oder
- insbesondere Altlasten aus Abfalldeponien umfassen, und/oder
dass das organik- und/oder wasserhaltige Stoffgemisch (2) insbesondere aus Biomassen zusammengesetzt ist, welche insbesondere land- und forstwirtschaftliche Biomassen umfassen.

4. Verfahren nach Anspruch 1, **dadurch gekennzeichnet, dass** eine Entwässerung des Stoffgemisches (2) in der Prozessstufe (4) mittels Entwässerungseinrichtung 12 auch ohne vorhergehende Auswaschung in der ersten Prozessstufe (3) erfolgt.

5. Verfahren nach Anspruch 1, **dadurch gekennzeichnet, dass** in der ersten Prozessstufe (3) mittels der Auswasch- und Dosiereinrichtung (7) die Auswaschung durch ein intensives Abwaschen und/oder Auswaschen auch unter Einstauung des Prozesswassers (10) lösliche organische und anorganische Bestandteile aus dem Stoffgemisch (2) in das Prozessproduktwasser (11) überführt.

6. Verfahren nach Anspruch 1, **dadurch gekennzeichnet, dass** in der ersten Prozessstufe (3) durch die Auswasch- und Dosiereinrichtung (7) eine kontinuierliche und /oder diskontinuierliche Durchmischung und/oder Umwälzung des Stoffgemisches (2) erfolgen.

7. Verfahren nach einem der vorhergehenden Ansprüche, **dadurch gekennzeichnet, dass** im Wasserpfad mit dem Prozessproduktwasser (11, 14) eine mechanische Prozessproduktwasseraufbereitung (5) zur Abtrennung von biologisch nicht oder schwer abbaubaren Feststoffen (18) insbesondere mittels Sieb- und Trenneinrichtung für Grob- und Faserstoffe (17) und/oder insbesondere mittels Sandfang (16) erfolgen.

8. Verfahren nach einem der vorhergehenden Ansprüche, **dadurch gekennzeichnet, dass** das Prozessproduktwasser (21) in einer insbesondere biologischen und/oder in einer insbesondere chemisch-physikalischen Prozessproduktwasseraufbereitung (6) behandelt wird.

9. Verfahren nach einem der vorhergehenden Ansprüche, **dadurch gekennzeichnet, dass** das Prozesswasser (22) insbesondere aus einer biologischen und/oder insbesondere aus einer chemisch-physikalischen Prozessproduktwasser-aufbereitung (6) einer Erwärmung (23) mindestens 35 °C, vorzugsweise 40-70°C unterzogen wird.

10. Verfahren nach einem der vorhergehenden Ansprüche, **dadurch gekennzeichnet, dass** das Prozesswasser (22) insbesondere vollständig oder insbesondere wenigstens teilweise wieder als Prozesswasser (10) der ersten Prozessstufe (3) zurückgeführt wird.

11. Verfahren nach einem der vorhergehenden Ansprüche, **dadurch gekennzeichnet, dass** das Stoffgemisch (2; 2.1) ohne mechanische Vorbehandlung insbesondere in der Prozessstufe (3) und/oder insbesondere in der Prozessstufe (4) behandelt wird.

12. Verfahren nach einem der vorhergehenden Ansprüche, **dadurch gekennzeichnet, dass** das Stoffgemisch (2; 2.2) einer mechanischen Vorbehandlung beispielsweise mittels insbesondere Dekompaktierung und/oder insbesondere Zerkleinerung und/oder insbesondere Siebung und/oder insbesondere Entnahme von Wert- und Störstoffen unterzogen wird, bevor das Stoffgemisch (2; 2.2) insbesondere in der Prozessstufe (3) und/oder insbesondere in der Prozessstufe (4) behandelt wird.

13. Verfahren nach einem der vorhergehenden Ansprüche, **dadurch gekennzeichnet, dass** das Stoffgemisch (2; 2.3) einer biologischen Vorbehandlung beispielsweise insbesondere einer Kompostierung und/oder insbesondere Vergärung und/oder insbesondere Fermentation unterzogen wird, bevor das Stoffgemisch (2; 2.3) insbesondere in der Prozessstufe (3) und/oder insbesondere in der Prozessstufe (4) behandelt wird.

14. Anlage nach einem der vorhergehenden Ansprüche **dadurch gekennzeichnet, dass** in der ersten Prozessstufe (3) als Auswasch- und Dosiereinrichtung (7) insbesondere eine und/oder insbesondere mehrere insbesondere nebeneinander und/oder insbesondere hintereinander angeordnete inbesondere Schnecken und/oder insbesondere Spiralen verwendet werden.

15. Anlage nach einem der vorhergehenden Ansprüche **dadurch gekennzeichnet, dass** das zu behandelnde Stoffgemisch (2) insbesondere kontinuierlich und/oder insbesondere diskontinuierlich der zweiten Prozessstufe (4) zugeführt wird, wobei die Verweildauer des Stoffgemisches (2) in der ersten Prozessstufe (4) zwischen 0,1 bis 24 Stunden und vorzugsweise 2 bis 6 Stunden beträgt.

16. Anlage nach einem der vorhergehenden Ansprüche **dadurch gekennzeichnet, dass** die Auswasch- und Dosiereinrichtung (7) in der ersten Prozessstufe (3) über eine Siebeinrichtung (8) mittels insbesondere Spalt- und/oder insbesondere Loch- und/oder insbesondere Ringsieb mit einem Siebschnitt von insbesondere vorzugsweise 5 - 12 mm verfügt, um eine Vorentwässerung in der ersten Prozessstufe vorzunehmen.

17. Anlage nach einem der vorhergehenden Ansprüche **dadurch gekennzeichnet, dass** als mechanisch wirkende Entwässerungseinrichtung (12) beispielsweise insbesondere eine Schneckenpresse oder/und insbesondere ein Separator, oder/und insbesondere eine insbesondere hydraulisch- oder insbesondere druckluftbetriebene Entwässerungspresse verwendet wird.
